# EUROPÄISCHE PATENTSCHRIFT

(11) **EP 1 534 283 B1**
(45) Veröffentlichungstag und Bekanntmachung des Hinweises auf die Patenterteilung: **14.12.2005**
(21) Anmeldenummer: 03771071.2
(22) Anmeldetag: 22.07.2003
(51) Int. Cl.: A61K 31/495, A61K 9/127, A61P 35/00, C07D 295/18

(54) **LIPOSOMALE FORMULIERUNGEN VON PHENYLALANIN-DERIVATEN**
FORMULATION OF LIPOSOMAL DERIVATIVES OF PHENYLALANINE
FORMULATIONS LIPOSOMALES DE DERIVES DE PHENYLALANINE

(30) Priorität: 24.07.2002 DE 10233632
(43) Veröffentlichungstag der Anmeldung: 01.06.2005
(73) Patentinhaber: Wilex AG, 81675 München (DE)
(72) Erfinder: WOSIKOWSKI-BUTERS, Katja, 85586 Poing (DE); SCHMALIX, Wolfgang, 82194 Gröbenzell (DE)
(74) Vertreter: Weiss, Wolfgang
(86) Internationale Anmeldenummer: PCT/EP2003/008011
(87) Internationale Veröffentlichungsnummer: WO 2004/011004

(56) Entgegenhaltungen:
- WO-A-00/04954
- WO-A-01/70204
- DE-A- 19 940 389
- STURZEBECHER J ET AL: "3-Amidinophenylalanine-based Inhibitors of Urokinase" BIOORGANIC & MEDICINAL CHEMISTRY LETTERS, OXFORD, GB, Bd. 9, Nr. 21, 1. November 1999 (1999-11-01), Seiten 3147-3152, XP004181024 ISSN: 0960-894X

## Beschreibung

Die Erfindung betrifft pharmazeutische liposomale Formulierungen von Phenylalanin-Derivaten und deren Verwendung als Urokinase-Inhibitoren, insbesondere zur Behandlung von malignen Tumoren und von Tumormetastasen.

Die Fähigkeit solider Tumoren zur Ausbreitung und Metastasierung in umgebendes Gewebe korreliert mit dem Abbau bzw. Umbau der extrazellulären Matrix (Tumorstroma) in der Umgebung der Tumorzelle bzw. mit deren Fähigkeit zur Durchdringung der Basalmembran. Obwohl die (patho)biochemischen Zusammenhänge noch nicht endgültig aufgeklärt sind, kommen dem Plasminogenaktivator Urokinase (uPA) und dem Urokinaserezeptor (uPAR) eine zentrale Bedeutung zu. uPA vermittelt die proteolytische Spaltung von Plasminogen zu Plasmin. Plasmin wiederum ist eine Protease mit breitem Wirkspektrum, die Komponenten der extrazellulären Matrix wie Fibrin, Fibronektin, Laminin und das Proteingerüst der Proteoglykane direkt abzubauen vermag. Außerdem kann Plasmin "latente" Metalloproteasen und das inaktive Proenzym von uPA, pro-uPA, aktivieren.

Tumorzellen und nichtmaligne Zellen des Tumorstromas synthetisieren und sezernieren das enzymatisch inaktive Proenzym pro-uPA. Proteasen, wie z.B. Plasmin oder die Kathepsine B und L, spalten pro-uPA durch limitierte Proteolyse zur aktiven Serinprotease HMW-uPA (HMW=high molecular weight). pro-uPA und die aktive Protease HMW-uPA binden an den Zeiloberflächenrezeptor uPAR (CD87). Plasmin(ogen) bindet ebenfalls an spezifische Rezeptoren auf der Plasmamembran der Tumorzelle, wodurch eine Fokussierung und Amplifikation der Plasminogenaktivierung in der direkten Umgebung der Tumorzelle erreicht wird. Invasiven Zellen ist somit die Möglichkeit gegeben, die extrazelluläre Matrix abzubauen, ohne sich der für eine gerichtete Bewegung notwendigen Unterlagen durch Proteolyse zu entziehen.

In verschiedenen zellbiologischen Studien konnte gezeigt werden, dass dem zellassoziierten Plasminogenaktivator-System innerhalb der kaskadenartigen Reaktionswege tumorassoziierter Proteolysesysteme ein besonderer Stellenwert zukommt (Wilhelm et al. (1994) The Urokinase/Urokinase receptor system: A new target for cancer therapy? In: Schmitt M., Graeff H., Kindermann G. (Hrsg): Prospects in Diagnosis and Treatment of Cancer. International Congress Series, Excerpta Medica 1050, Amsterdam, Elsevier 1994, pp145-156). An Kulturen humaner Kolonkarzinomzellen wurde beobachtet, dass deren Fähigkeit, eine extrazelluläre Matrix zu durchwandern, vom Sättigungsgrad der uPA-Rezeptoren mit aktivem uPA abhängig ist (Hollas et al., Cancer Res. 51 (1991), 3690-3695). Ebenfalls im Zellkulturmodell wurde eine Reduktion des invasiven Potenzials von Zellen beobachtet, wenn die proteolytische Aktivität von uPA durch PAI-1 (Cajot et al., Proc. Natl. Acad. Sci. USA 87 (1990), 6939-6943) oder PAI-2 (Baker et al., Cancer Res. 50 (1990), 4676-4684) gehemmt wurde. Ein vergleichbarer Effekt wurde bei Hemmung der Bindung von uPA an die Zelloberfläche durch Blockierung des Rezeptors mittels proteolytisch inaktiver uPA-Varianten erzielt (Cohen et al., Blood 78 (1991), 479-487; Kobayashi et al., Br. J. Cancer 67 (1993), 537-544). Auch die Transfektion epidermoider Karzinomzellen mit einem Plasmid, das ein Antisense-Transkript gegen einen Teil von uPAR exprimiert, führte durch Unterdrückung der uPAR-Synthese zur Verringerung der Invasivität dieser Zellen (Kook, EMBO J. 13 (1994), 3983-3991). Gegen uPA und PAI-1 gerichtete Antikörper reduzierten das invasive Potential von Lungenkrebszellen *in vitro* (Liu et al., Int. J. Cancer 60 (1995), 501-506).

Der Einfluss des Plasminogenaktivator-Systems auf den Metastasierungsprozess konnte auch in Tumor-Tiermodellen belegt werden.

So wurde die durch menschliche Karzinomzellen verursachte Bildung von Lungenmetastasen in Hühnerembryos durch Zugabe von Antikörpern gegen uPA fast vollständig verhindert (Ossowski und Reich, Cell 35 (1983), 611-619). Metastasierende menschliche Karzinomzellen wurden mit einem Expressionsplasmid transfiziert, das für eine proteolytisch inaktive, aber uPAR-bindende uPA-Mutante kodierte. Im Mausmodell zeigte sich, dass die Karzinomzellen, die inaktives uPA synthetisierten, nach Injektion im Vergleich zu den nichttransfizierten Zellen eine signifikant geringere Anzahl an Metastasen bildeten (Crowley et al., Proc. Natl. Acad. Sci. USA 90 (1993), 5021-5025). Nach Verabreichung von uPA-Antisense Oligonukleotiden wurde darüber hinaus eine Inhibierung der intraperitonealen Ausbreitung von humanen Ovarialkarzinomzellen in Nacktmäusen beobachtet (Wilhelm et al., Clin. Exp. Metast. 13 (1995), 296-302).

In den letzten Jahren wurde die klinische Relevanz von Faktoren des Plasminogenaktivator-Systems (uPA, uPAR, PAI-1 und PAI-2) für die Prognose von Patienten mit soliden malignen Tumoren intensiv untersucht. Dabei erwies sich der uPA-Antigengehalt bei verschiedenen Tumoren (z.B. Brust, Eierstock, Magen, Lunge, Niere etc.) sowohl für das rezidivfreie Überleben als auch für das Versterben als ein starker Prognosefaktor (siehe beispielsweise Schmitt et al., J. Obstet. Gynaecol. 21 (1995), 151-165; Jaenicke et al., Breast Cancer Res. Treat. 24 (1993), 195-208; Kuhn et al., Gynecol. Oncol. 55 (1994), 401-409; Nekarda et al., Lancet 343 (1994), 117; Pedersen et al., Cancer Res. 54 (1994), 4671-4675). Ebenso korrelieren erhöhte Konzentrationen an uPAR in Lungen- (Pedersen et al., supra) und Brustkrebsgewebe (Duggan et al., Int. J. Cancer 61 (1995), 597-600; Ronne et al., Breast Cancer Res. Treat. 33 (1995), 199-207) sowie bei Magenkrebs, sowohl im Tumorgewebe selbst (Heiss et al., J. Clin. Oncol. 13 (1995), 2084-2093) als auch bei den ins Knochenmark ausgestreuten Tumorzellen (Heiss et al., Nature Medicine 1 (1995), 1035-1039) mit einer schlechten Prognose.

Es wurde auch gefunden, dass an Position 2 mit einem Phenylrest substituierte 3-Amidinophenylalanin-Derivate selektive und *in vivo* wirksame Hemmstoffe von uPA darstellen (PCT/EP99/05145). Die Verabreichung dieser Verbindungen im Tierexperiment erfolgt in Form von wässrigen Lösungen. DE 102 25 876.7 offenbart die Verwendung von 3-Guanidinophenylalanin-Derivaten als Urokinase-Inhibitoren.

Im Rahmen der ersten klinischen Erprobungen der oben genannten Verbindungen hat sich gezeigt, dass die Verabreichung in Form von wässrigen Lösungen mit Nachteilen behaftet ist. So wurde festgestellt, dass die Verbindungen in höher konzentrierten Lösungen bei intravenöser Injektion oder Infusion hämolytische Eigenschaften besitzen und bei subkutaner Verabreichung zu Hautreizungen führen. Zur Verabreichung von gering konzentrierten Lösungen werden jedoch große Infusionsvolumina benötigt und eine subkutane Verabreichung wirksamer Mengen des Mittels in Form wässriger Lösungen ist nicht möglich.

Es bestand daher das Bedürfnis, neue pharmazeutische Formulierungen der Phenylalanin-Derivate zu entwickeln, die einerseits stabil sind und eine hohe Wirksamkeit besitzen, aber andererseits nicht zu unerwünschten Nebenwirkungen, wie Hämolyse oder Hautreizungen, führen.

Versuche zur Stabilisierung von wässrigen Lösungen durch Zusatz oberflächenaktiver Mittel, wie etwa Pluronic F68 oder Tween 80, bzw. Stabilisatoren, wie Humanserumalbumin, waren jedoch erfolglos. Auch der Zusatz von Cosolventien, wie Polyethylenglycolen, führte nicht zum gewünschten Ergebnis. Schließlich konnte auch durch Formulierung des Wirkstoffs in gemischten Micellen, die das Gallensalz Glycocholatmonohydyrat und das Phospholipid Ei-Phosphatidylcholin enthielten, nicht für eine ausreichende Stabilität gesorgt werden.

Die der Erfindung zugrunde liegende Aufgabe bestand somit darin, pharmazeutische Formulierungen mit einem von 3-Amidinophenylalanin bzw. 3-Guanidinophenylalanin abgeleiteten Urokinase-Inhibitor bereitzustellen, die einerseits eine hohe Wirksamkeit besitzen und andererseits stabil und verträglich sind.

Insbesondere betrifft die vorliegende Erfindung von 3-Amidinophenylalanin oder 3-Guanidinophenylalanin abgeleitete neue Urokinase-Inhibitoren der allgemeinen Formel I, 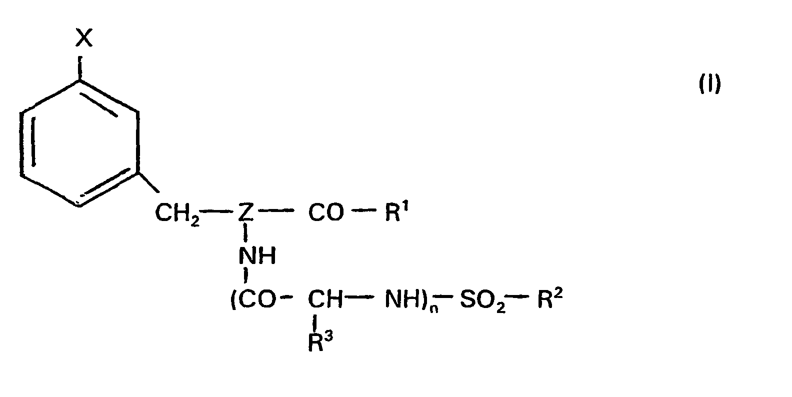 die als Racemate sowie als L- bzw. D-konfigurierte Verbindungen vorliegen und in denen
- X: eine Amidino- oder Guanidinogruppe ist,
- R1: (a) OH oder OR⁴ ist, wobei R⁴ ein gegebenenfalls z.B. mit Hydroxyl, Carboxyl, Sulfonyl, Nitro, Cyano, Oxo oder/und Halogen substituiertes, verzweigtes oder unverzweigtes C₁-C₈-Alkyl, C₃-C₈-Cycloalkyl oder Aralkyl, z.B. Benzyl oder Phenylethyl ist,
(b) eine Gruppe der Formel 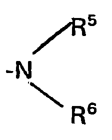 darstellt, in welcher R⁵ und R⁶ beliebige mit der Gesamtstruktur kompatible Reste sind, wobei insbesondere
   (i) R⁵ und R⁶ H sind,
   (ii) R⁵ H ist und R⁶ ein gegebenenfalls z.B. mit Hydroxyl, Carboxyl, Sulfonyl, Nitro, Cyano, Oxo oder/und Halogen substituiertes verzweigtes oder unverzweigtes C₁-C₈-Alkyl, Aralkyl, z.B. Benzyl oder Phenylethyl, oder C₅-C₈ Cycloalkyl ist,
   (iii) R⁵ und R⁶ jeweils unabhängig ein gegebenenfalls z.B. mit Hydroxyl oder/und Halogen substituiertes, unverzweigtes oder verzweigtes C₁-C₄ Alkyl sind oder
   (iv) R⁵ H ist und R⁶ -NH₂ oder eine insbesondere mit Aryl oder Heteroaryl substituierte Aminogruppe ist,
   (v) R⁵ H oder ein gegebenenfalls z.B. mit Hydroxyl oder/und Halogen substituiertes, unverzweigtes oder verzweigtes C₁-C₄ Alkyl ist und R⁶ der Rest einer Aminosäure, z.B. einer α-, β- oder ω-Aminocarbon-oder Aminosulfonsäure, oder der Rest eines Peptids z.B. mit einer Länge bis zu 50 Aminosäuren oder eines Polypeptids z.B. mit einer Länge von mehr als 50 Aminosäuren bis 1.000 Aminosäuren ist,
(c) eine Gruppe der Formel 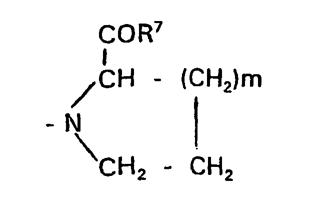 darstellt, in welcher m die Zahl 1 oder 2 bezeichnet, und in welcher eine oder mehrere der Methylengruppen gegebenenfalls z.B. mit einem Hydroxyl-, Carboxyl-, C₁-C₄ Alkyl- oder Aralkylrest, z.B. Benzyl oder Phenylethyl, substituiert sind, wobei die Gruppe (c) racemisch oder 0- bzw. L-konfiguriert ist, und R⁷ die Bedeutung von R¹ in den Ziffern (a), (b) und (f) aufweist,
(d) eine Gruppe der Formel 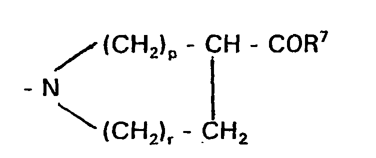 darstellt, in welcher p = r = 1, p = 1 und r = 2 oder p = 2 und r = 1 sind und in welcher eine oder mehrere der Methylengruppen gegebenenfalls z.B. mit einem Hydroxyl-, Carboxyl-, C₁-C₄-Alkyl- oder Aralkylrest, z.B. Benzyl oder Phenylethyl, substitutiert sind, und R⁷ die Bedeutung von R¹ in Ziffer (a), (b) und (f) aufweist,
(e) eine Piperidylgruppe darstellt, die gegebenenfalls in einer der Stellungen 2, 3 und 4 z.B. mit einem C₁-C₄-Alkyl- , C₁-C₃-Alkoxy- oder Hydroxylrest substituiert ist,
   wobei gegebenenfalls an die heterocycloaliphatischen Ringe der Formeln (c), (d), (e) ein weiterer aromatischer oder cycloaliphatischer Ring, vorzugsweise Phenyl oder Cyclohexyl, in 2,3 oder 3,4 Stellung, bezogen auf das Heteroatom, ankondensiert ist,
(f) eine Gruppe der Formel 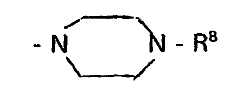 darstellt, in welcher R⁸
   (i) einen gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituierten C₁-C₆-Alkylrest, wie z.B. Ethoxycarbonyl, oder Arylrest, wie z.B. Phenyl, p-Halogenphenyl, Naphthyl,
   (ii) einen gesättigten oder ungesättigten, verzweigten oder unverzweigten-C₁-C₆-Alkoxyrest oder
   (iii) einen gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituierten Phenoxy- bzw. Benzyloxycarbonylrest bedeutet,
(g) einen Acylrest der Formel -COX darstellt, wobei X
   (i) H, einen gegebenenfalls z.B. mit Hydroxyl, Carboxyl, Sulfonyl, Nitro, Cyano, Oxo oder/und Halogen substituierten, unverzweigten oder verzweigten Alkylrest, vorzugsweise einen C₁-C₆-Alkylrest, insbesondere Methyl,
   (ii) einen gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituierten Aryl- oder Heteroarylrest, wie z.B. Phenyl, p-Halogenphenyl, Thienyl oder
   (iii) einen gegebenenfalls z.B. mit Hydroxyl, Carboxyl, Sulfonyl, Nitro, Cyano, Oxo oder/und Halogen substituierten Cycloalkylrest, vorzugsweise einen C₃-C₁₀-Cycloalkylrest bedeutet,
(h) einen Aralkylrest, z.B. Benzyl oder Phenylethyl, darstellt, in dem der aromatische Rest gegebenenfalls z.B. mit einem Halogenatom, einer C₁-C₆-Alkyl-, C₁-C₃-Alkoxy-, Hydroxy-, Cyano-, Carboxyl-, Sulfonyl- oder Nitrogruppe substituiert ist,

(i) einen Carbonsäureamidrest der Formel -CONR'R", einen Thiocarbonsäureamidrest -CSNR'R" oder einen Essigsäureamidrest -CH₂-CONR'R" darstellt, wobei
   (i) R' und R" H sind,
   (ii) R' und R" jeweils unabhängig C₁-C₄-Alkyl sind,
   (iii) R' H ist und R" C₁-C₄-Alkyl ist,
   (iv) R' H ist und R" Aryl, z.B. Phenyl, ist oder
   (v) R' und R" mit dem Stickstoffatom einen heterocycloaliphatischen Ring mit 5-7 Ringgliedern, der ein weiteres Heteroatom, z.B. N, O oder/und S tragen kann, bilden,
(j) einen SO₂-Y-Rest darstellt, in dem Y
   (i) ein gegebenenfalls z.B. mit Hydroxyl, Carboxyl, Sulfonyl, Nitro, Cyano, Oxo oder/und Halogen substitutiertes C₁-C₈-Alkyl, vorzugsweise Methyl, Trifluormethyl, Trichlormethyl,
   (ii) ein gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituiertes Aryl oder Heteroaryl, wie z.B. Phenyl, 4-Methyl-phenyl, 2,4,6-Trimethyl-phenyl, 2,4,6-Triisopropyl-phenyl, 4-Methoxy-2,3,6-Trimethyl-phenyl, 2,2-Dimethyl-6-methoxy-chromanyl, 2,2,5,7,8-Pentamethyl-chromanyl, Anthrachinonyl, Naphthyl oder Chinolyl, bzw. O-Aryl, vorzugsweise O-Phenyl, oder O-Heteroaryl oder
   (iii) -NR'R" ist, wobei R' und R" jeweils unabhängig H oder C₁-C₃-Alkyl bedeuten,
(k) einen cycloaliphatischen Ring mit 5 bis 8 C-Atomen darstellt, der gegebenenfalls z.B. mit einer C₁-C₆-Alkyl-, C₁-C₃-Alkoxy-, Halogen-, Hydroxyl- oder/und Oxogruppe substituiert ist,
(I) einen gegebenenfalls z.B. mit C₁-C₆-Alkyl, C₁-C₃-Alkoxy, Hydroxyl, Carboxyl, Sulfonyl, Nitro, Cyano, Oxo oder/und Halogen substituierten Heteroarylrest, wie z.B. Pyridyl oder Pyrimidyl, oder heterocycloaliphatischen Rest, beispielsweise N-Methylpiperidyl darstellt,
(m) einen funktionalisierten Alkylrest der Formel -(CH₂)ₙ-X darstellt, wobei die Alkylkette unverzweigt oder verzweigt ist, n = 1 bis 8 bedeutet und der funktionelle Rest X
   (i) eine Hydroxylgruppe darstellt, deren H-Atom gegebenenfalls durch eine C₁-C₄-Alkyl-, Aralkyl-, z.B. Benzyl oder Phenylethyl, Aryl-, z.B. Phenyl, C₁-C₄-Hydroxyalkyl- oder Acylgruppe CO-Alkyl, (C₁-C₆), substituiert ist,
   (ii) ein Halogenatom bedeutet,
   (iii) eine tertiäre Aminogruppe der Formel -N(Alk)₂ darstellt, wobei die Alkylgruppen 1 bis 3 C-Atome sowie vorzugsweise die gleiche Bedeutung besitzen und das Stickstoffatom gegebenenfalls einem heterocycloaliphatischen Ring mit 5-7 Ringgliedern, der ein weiteres Heteroatom, z.B. N, O oder/und S tragen kann, angehört,
- R²: einen gegebenenfalls z.B. mit C₁-C₆ Alkyl, C₁-C₃-Alkoxy, Hydroxyl, Carboxyl, Sulfonyl, Nitro, Cyano, Oxo oder/und Halogen substituierten Phenylrest, wie beispielsweise Phenyl, 4-Methyl-phenyl, 2,4,6-Trimethyl-phenyl, 2,4,6-Triisopropyl-phenyl, 4-Methoxy-2,3,6-trimethyl-phenyl darstellt,
- R³: H oder verzweigtes bzw. unverzweigtes C₁-C₄-Alkyl ist und n 0 oder 1 bedeutet,
- Z: N oder CR⁹ bedeutet, wobei R⁹ H oder verzweigtes oder unverzweigtes C₁-C₄-Alkyl ist.

Die Verbindungen können auch als Salze, vorzugsweise als physiologisch verträgliche Säuresalze, z.B. als Salze von Mineralsäuren, besonders bevorzugt als Hydrochloride, oder als Salze von geeigneten organischen Säuren vorliegen.

Von den in den allgemeinen Ansprüchen definierten Verbindungen sind solche, bei denen R¹ einer Gruppe der Formeln (b), (d) und (f) entspricht, R² einen einfach, zweifach oder dreifach alkylsubstituierten Phenylrest, insbesondere einen 2,4,6-substituierten Phenylrest, z.B. einen 2,4,6-Trüsopropylphenyl-Rest darstellt, und n = 0 ist, von besonderer Bedeutung. Weiterhin bevorzugt sind Verbindungen, bei denen Z CH oder N ist.

Besonders bevorzugt handelt es sich bei der Verbindung der Formel (I) um Nα-(2,4,6-Triisopropyl-phenylsulfonyl)-3-amidino-(D,L)-phenylalanin-4-ethoxycarbonyl-piperazid, Nα-(2,4,6-Triisopropyl-phenylsulfonyl)-3-guanidino-(D,L)-phenylalanin-4-ethoxycarbonyl-piperazid bzw. um das L-Enantiomere davon oder um ein pharmazeutisch verträgliches Salz dieser Verbindungen.

Die erfindungsgemäßen Verbindungen sind in der Lage, hocheffizient das Wachstum oder/und die Ausbreitung von malignen Tumoren zu hemmen, z.B. die Tumorausbreitung beim Pankreaskarzinom, das Tumorwachstum des Mammakarzinoms sowie die Metastasierung von Tumoren. Dabei können die uPA-Inhibitoren gegebenenfalls zusammen mit anderen Antitumormitteln oder mit anderen Behandlungsarten, z. B. Bestrahlung oder chirurgischen Eingriffen, eingesetzt werden. Weiterhin sind die erfindungsgemäßen Inhibitoren auch für andere uPA-assoziierte Erkrankungen wirksam (z.B. bei der Verhinderung der Blasenbildung bei der Hauterkrankung Pemphigus vulgaris).

Die erfindungsgemäßen liposomalen Formulierungen enthalten den Wirkstoff vorzugsweise in einem Gewichtsanteil von 0,5-10 %, besonders bevorzugt von 2-5 %, bezogen auf das Gesamtgewicht der Formulierung. Weiterhin ist bevorzugt, dass die Formulierung - zumindest deren wässrige Komponente - einen pH-Wert im Bereich von 5,5-9,0 aufweist. Der Gehalt an freiem, d.h. nach Abfiltration von Lipidkomponenten in der wässrigen Phase der Formulierung vorliegendem Wirkstoff, ist vorzugsweise ≤ 1 mg/ml, besonders bevorzugt ≤ 500 µg/ml und am meisten bevorzugt ≤ 100 *µ*g/ml.

Die Formulierung enthält vorzugsweise Phospholipide in einem Gewichtsanteil von 4,5-40 %, besonders bevorzugt von 6-15 %, bezogen auf das Gesamtgewicht der Formulierung. Beispiele für geeignete Phospholipide sind neutrale Phospholipide, wie etwa Phosphatidylcholin, anionische Phospholipide, wie etwa Phosphatidylethanolamin, Phosphatidylglycerin, Diphosphatidylglycerin (Cardiolipin), Phosphoinositol und veresterte Derivate davon, wie etwa Dimyristoylphosphatidylglycerin. Es können z.B. Phospholipide aus natürlichen Quellen, z.B. Ei- oder Sojalecithin, synthetische Phospholipide oder Kombinationen davon verwendet werden. Besonders bevorzugt enthält die Formulierung mindestens ein anionisches Phospholipid. Gute Ergebnisse wurden erhalten, wenn man eine Kombination von Phosphatidylcholin und Dimyristoylphosphatidylglycerin in einem Gewichtsverhältnis von 70:30 verwendet.

Weiterhin kann die Formulierung vorzugsweise eine membranstabilisierende Komponente enthalten, wie etwa Cholesterin oder Derivate davon, in einem Gewichtsanteil von bis zu 5 %, bezogen auf das Gesamtgewicht der Formulierung. Die Membranstabilität bzw. -rigidität kann aber auch durch Auswahl entsprechender Fettsäuren in den Phospholipiden oder gegebenenfalls anderen Lipidkomponenten über die Kettenlänge oder/und den Grad der Unsättigung eingestellt werden.

Vorzugsweise enthält die Formulierung auch ein Gefrierschutzmittel, das günstigerweise in einem Gewichtsanteil von bis zu 15 %, vorzugsweise von 5-15 %, bezogen auf das Gesamtgewicht der Formulierung vorliegt. Beispiele für geeignete Gefrierschutzmittel sind Kohlenhydrate, z.B. Mono-, Di- oder Trisaccharide, wie etwa Lactose, Saccharose, Trilactose, Maltose, Trehalose etc. oder/und Zuckeralkohole, wie etwa Sorbit, Mannit etc.

Die erfindungsgemäße liposomale Formulierung kann unilamellare Liposomen, multilamellare Liposomen, ungeordnete Komplexe und Kombinationen davon enthalten. Bevorzugt sind Liposomen, bei denen der mittlere Durchmesser nach Herstellung nicht größer als 500 nm ist und insbesondere im Bereich von 100-250 nm liegt.

Die erfindungsgemäße liposomale Formulierung kann beispielsweise hergestellt werden durch Hochdruckhomogenisierung einer den Wirkstoff und die Lipide enthaltenden Suspension und gegebenenfalls anschließende Filtration. Andere Möglichkeiten sind dem Fachmann bekannt, z.B. Lösungsmittelinjektion, Hydratation von Lipidfilmen etc.

Die liposomale Formulierung kann sterilfiltriert und gegebenenfalls dehydratisiert werden, z.B. durch Lyophilisierung. Nach wässriger Rekonstitution von dehydratisierten Liposomen kann sich der mittlere Durchmesser erhöhen, z.B. auf 100-1.000 nm.

Die erfindungsgemäße Formulierung kann zur parenteralen Applikation, z.B. zur intravenösen Injektion, zur Infusion oder zur subkutanen oder intramuskulären Injektion, eingesetzt werden. Die Tagesdosis ist vorzugsweise 5-250 mg, besonders bevorzugt von 20-120 mg bei subkutaner oder intramuskulärer Verabreichung und von 10-500 mg, besonders bevorzugt von 50-250 mg bei intravenöser Verabreichung, jeweils bezogen auf ein mittleres Körpergewicht von 70 kg. Die Verabreichung erfolgt vorzugsweise einmal täglich bis einmal wöchentlich.

Die erfindungsgemäße Formulierung kann gegebenenfalls in Kombination mit anderen Wirkstoffen, z.B. cytotoxischen Mitteln, eingesetzt werden. Die Verabreichung erfolgt vorzugsweise gemeinsam, z.B. als Vor- oder/und Nachbehandlung im Zusammenhang mit chirurgischen Eingriffen, Strahlenbehandlung oder/und Chemotherapie.

Weiterhin soll die Erfindung an den folgenden Beispielen näher erläutert werden.

### Beispiel 1: Herstellung von liposomalen Präparationen

### 1.1 Materialien

Dimyristoylphosphatidylglycerin-Natriumsalz (DMPG-Na) wurde von Nippon Fine Chemicals bezogen. Ei-Phosphatidylcholin (EPC) wurde von Lipoid KG bezogen.

### 1.2 Herstellung von Liposomen mit pH 6,5

Die Phospholipide und der Wirkstoff Nα-(2,4,6-Triisopropylphenylsulfonyl)-3-amidino-(L)-phenylalanin-4-ethoxycarbonylpiperazid (WX-UK1) als Hydrochlorid wurden zusammen in 50 mmol/l Phosphatpuffer pH 6,5 und 9 % Lactose dispergiert. Anschließend wurde das Gemisch auf 40 °C erhitzt und für 30 min in ein Ultraschallbad gegeben.

Zur Hochdruckhomogenisierung wurde ein EmulsiFlex C5-Gerät von Avestin, ausgestattet mit einer Extrusionseinheit verwendet, so dass Homogenisierung und Hockdruckextrusion durch ein Polycarbonat-Membranfilter in Serie durchgeführt werden konnten.

Zunächst wurde die Liposomensuspension durch den Hockdruckhomogenisator für 6 min (entsprechend 20 Passagen) bei einem Druck von 345 bar geleitet. Dann wurde die Formulierung 6 min durch ein 100 nm Polycarbonat-Membranfilter in der Extrusionseinheit geleitet.

### 1.3 Herstellung von Liposomen mit pH 8,4

Liposomale Präparationen mit einem pH-Wert von 8,4 wurden auf ähnliche Weise erhalten. Zuächst wurde jedoch eine Dispersion mit einem pH-Wert von 5,2 hergestellt und einer Hochdruckhomogenisierung unterzogen. Danach wurde durch Zugabe von Dinatriumhydrogenphosphat der pH-Wert auf 8,4 eingestellt und eine weitere Homogenisierung für 6 min bei einem Druck von 345 bar durchgeführt.

### 1.4 Sterilfiltration, Abfüllung und Lyophilisierung

Die liposomalen Formulierungen wurden durch ein sterilisiertes 450 nm Filter (Polyvinylidendifluorid-Durapore von Millipore) filtriert, die filtrierten Suspensionen in 0,5 ml Aliquots abgefüllt und dann lyophilisiert.

### 1.5 Größenbestimmung

Zur Bestimmung der mittleren Größe und der Größenverteilung der liposomalen Suspensionen wurde ein Malvern Zetasizer 1000 verwendet. Der Homogenitätsgrad der Suspensionen wurde als Polydispersitätsindex (PI) ausgedrückt. Ein Wert von 0,1 bedeutet eine sehr enge Größenverteilung und ein Wert von 0,9 eine sehr breite Verteilung.

### 1.6 Resultate

Die Zusammensetzungen und die Analysenergebnisse sind in der nachfolgenden **Tabelle 1** zusammengefasst. Nach Rekonstitution war der Wirkstoffgehalt der liposomalen Formulierung zwischen 18 und 21 mg/ml und die Teilchengröße zwischen 350 und 500 nm, wobei die Teilchengröße während der Lyophilisierung und Rekonstitution signifikant zunahm.

Liposomen, die Wirkstoff zusammen mit negativ geladenen Phospholipiden enthielten, konnten verdünnt werden, während neutrale Liposomen bei Zugabe von Puffer eine Präzipitation zeigten.

**Tabelle 1**

| **Komponenten** | **Konzentration (%)** | **Teilchengröße (nm) / PI** | | **Tatsächliche Konzentration nach Rekonstitution (%)** |
|---|---|---|---|---|
| | | **vor Rekonstitution** | **nach Rekonstitution** | |
| | | | | |

| **Formulierung 1** | | | | |
|---|---|---|---|---|
| **EPC pH 6,5** | | | | |
| WX-UK1.HCl | 2,00 | 108/0,27 | 518/0,53 | 1,98 |
| EPC | 10,00 | | | |
| Lactose | 7,91 | | | |
| Na₂HPO₄ x 2H₂O | 0,72 | | | |
| Wasser | ad 100 % | | | |
| | | | | |

| **Formulierung 2** | | | | |
|---|---|---|---|---|
| **PC/PG pH 6,5** | | | | |
| WX-UK1.HCl | 2,00 | 307/0,49 | 405/0,45 | 2,05 |
| EPC | 7,00 | | | |
| DMPG.Na | 3,00 | | | |
| Lactose | 7,90 | | | |
| Na₂HPO₄ x 2H₂O | 0,72 | | | |
| Wasser | ad 100 % | | | |
| | | | | |

| **Formulierung 3** | | | | |
|---|---|---|---|---|
| **EPC pH 8,4** | | | | |
| WX-UK1.HCl | 2,00 | 269/0,24 | 359/0,34 | 1,80 |
| EPC | 10,00 | | | |
| Lactose | 9,70 | | | |
| Na₂HPO₄ x 2H₂O | 0,70 | | | |
| Wasser | ad 100 % | | | |
| | | | | |

| **Formulierung 4** | | | | |
|---|---|---|---|---|
| **PC/PC pH 8,4** | | | | |
| WX-UK1.HCl | 2,00 | 202/0,23 | 353/0,43 | 1,98 |
| -EPC | 7,00 | | | |
| DMPG.Na | 3,00 | | | |
| Lactose | 9,70 | | | |
| Na₂HPO₄ x 2H₂O | 0,40 | | | |
| Wasser | ad 100 % | | | |

### Beispiel 2: Bioverfügbarkeit und Verträglichkeit liposomaler Formulierungen in der Ratte

### 2.1 Applikation

Weibliche Wistar Ratten, 240-300 g Körpergewicht (Charles River-Wiga, Sulzfeld) wurden mit dem Wirkstoff WX-UK1 in Form einer wässrigen Lösung oder mit den in Beispiel 1 beschriebenen liposomalen Formulierungen 1-4 behandelt. Die Tiere erhielten über einen Zeitraum von 10 Tagen jeweils 1 Gabe/Tag. Die Applikationen erfolgten in den Bereich der Flanke (zwischen Schulter und hinteren Gliedmaßen), so dass die Tiere die Injektionsstelle nicht mit den Pfoten erreichen konnten. Die Injektionsstellen wurden in Abständen von ca. 1 cm gesetzt, es wurde nicht mehrmals in die gleiche Stelle injiziert. Die ersten fünf Injektionen erfolgen auf der linken Seite, die zweiten fünf Injektionen auf der rechten Seite. Die Dosis betrug in der Regel 3 mg Wirkstoff/kg Körpergewicht, d.h.bei einem mittleren Körpergewicht von 300 g wurden 0,9 mg Wirkstoff appliziert. In einigen Fällen wurde radioaktiv markierter Wirkstoff (³H-WX-UK1) appliziert.

### 2.2 Beobachtung der Tiere

Die Tiere wurden vor jeder Injektion gewogen und nach jeder Injektion über 2 h beobachtet, vor jeder weiteren Injektion wurden die vorhergehenden Injektionsstellen palpiert. Das Verhalten der Tiere und der Palpationsbefund wurden protokolliert.

Zur Bewertung der beobachteten Beeinträchtigungen wurde folgender Score benutzt:

| Verhalten der Tiere nach der Injektion (2 h) | |
|---|---|
| unauffällig | 0 |
| leichtes Kratzen in 1.Stunde | 1 |
| Kratzen und Anzeichen von Schmerz | 2 |
| nach 2 h noch deutliche Beeinträchtigung | 3 |

| Laufender Palpationsbefund | |
|---|---|
| unauffällig | 0 |
| leichte, vereinzelte Verdickungen/Verhärtungen | 1 |
| bleibende Verdickungen/Verhärtungen, zusammenfließend | 2 |
| zusätzliche Läsionen, Entzündungen | 3 |

### 2.3 Untersuchungen nach Beendigung der Applikation

24 h nach der letzten Injektion wurden die Tiere narkotisiert (Ethylurethan, 1,4 g/kg i.p.) und aus dem retroorbitalen Venenplexus Citrat-Blut gewonnen; Citrat-Plasma wurde durch Zentrifugation bei 1.200 xg für 10 min erhalten. Dann wurde der Gallengang präpariert und über 15 min Galle gewonnen. Anschließend wurden die Hautbereiche der Injektionsstellen untersucht und bei der Präparation ein makroskopischer Befund erhoben.

Zur Bewertung der beobachteten makroskopischen Veränderungen wurde folgender Score benutzt:

| Abschließende Beobachtungen, makroskopischer Befund bei Präparation | |
|---|---|
| unauffällig | 0 |
| vereinzelte Verhärtungen bzw. schwartige Herde | 1 |
| zahlreiche schwartige Herde bzw. Knoten | 2 |
| zahlreiche schwartige, entzündliche Herde bzw. Knoten | 3 |

Von den Bereichen der Injektionen wurden Hautproben präpariert, in Formalin fixiert und histologisch untersucht (Hämatoxylin/Eosin-Färbung). Zur Bewertung der histologisch sichtbaren Veränderungen wurde folgender Score benutzt:

| Histologischer Befund: | |
|---|---|
| unauffällig | 0 |
| vereinzelte Nekrosen, vereinzelte entzündliche Reaktionen | 1 |
| Nekrosen oder entzündliche Reaktionen | 2 |
| Nekrosen und starke entzündliche Reaktionen und/oder weitere Veränderungen | 3 |

Anschließend wurden die Tiere getötet und verschiedene Organe (Herz, Niere, Leber, Milz) präpariert. In Plasma, Galle und den Organen wurde der Gehalt an Wirkstoff mittels HPLC [Standard-Methode mittels Nucleosil 7 C18 Säulen (Macherey-Nagel, Düren), Acetonitril/Wasser/Perchlorsäure 30/70/0,04, 1 ml/min] nach Vorreinigung mittels Chromabond C18 Festphasen-Extractionssäulen (Macherey-Nagel, Düren) bestimmt. Bei Einsatz von ³H-WX-UK1 wurde die Konzentration von Wirkstoff direkt nach Lyophilisation der Proben durch Messung der Radioaktivität in einem Flüssigkeits-Szintillationszähler bestimmt.

### 2.4 Ergebnisse

Als Kontrollen diente die Gabe von WX-UK1 gelöst in 0,5 ml NaCl (0,9 %) mit 5 % Ethanol (Versuche 628, 629)

Lyophilisierte neutrale Liposomen pH 6,5 (Formulierung 1) wurden mit Wasser resuspendiert und direkt eingesetzt. Entsprechend der dann enthaltenen Konzentration von 20 mg/ml wurden 45 *µ*l pro Ratte (300 g Körpergewicht) appliziert, das entspricht der gewünschten Dosis von 3 mg/kg Körpergewicht (Versuche 624, 625).

Lyophilisierte negative Liposomen pH 6,5 (Formulierung 2) wurden mit Wasser resuspendiert. Entsprechend der dann erhaltenen Konzentration von 16 mg/ml wurden 55 µl pro Ratte (300 g Körpergewicht) appliziert, das entspricht der gewünschten Dosis von 3 mg/kg Körpergewicht (Versuche 626, 627) .

Lyophilisierte neutrale Liposomen pH 8,4 (Formulierung 3) wurden mit Wasser suspendiert und direkt eingesetzt. Entsprechend der dann enthaltenen Konzentration von 20 mg/ml wurden 45 *µ*l pro Ratte (300 g Körpergewicht) appliziert, das entspricht der gewünschten Dosis von 3 mg/kg Körpergewicht (Versuche 632, 633).

Lyophilisierte negative Liposomen pH 8,4 (Formulierung 4) wurden mit Wasser suspendiert und direkt eingesetzt. Entsprechend der dann erhaltenen Konzentration von 20 mg/ml wurden 45 *µ*l pro Ratte (300 g Körpergewicht) appliziert, das entspricht der gewünschten Dosis von 3 mg/kg Körpergewicht (Versuche 637, 638).

### 2.5 Zusammenfassung der Ergebnisse

Die **Tabelle 2** enthält die Konzentrationen von WX-UK1 in Plasma, Galle und den Organen Niere, Leber, Milz und Herz. Die Konzentration im Plasma ließ sich nur bei Einsatz von ³H-WX-UK1 bestimmen, sie liegt bei < 100 ng/ml. Sehr vergleichbare Konzentrationen finden sich in den Kontrollen bei Applikationen in NaCl/Ethanol und Gabe der Liposomen 3 und 4. Bei den Liposomen 1 bzw. 2 wurden geringere Konzentrationen von WX-UK1 in Galle und Organen gefunden. Die Bewertung der histologischen Befunde ist in **Tabelle 2** zusammengestellt.

Die **Tabellen 3 und 4** enthalten die Bewertung der Appliktionsfolgen in den Tieren. Es ist zu erkennen, dass die Verabreichung der liposomalen Formulierungen zu erheblich geringeren Nebenwirkungen als die Verabreichung der wässrigen Lösung führt.

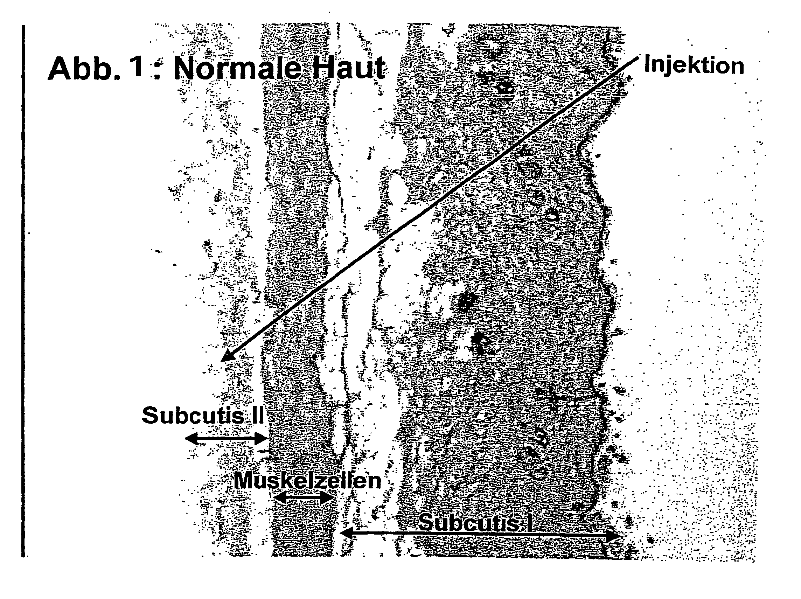

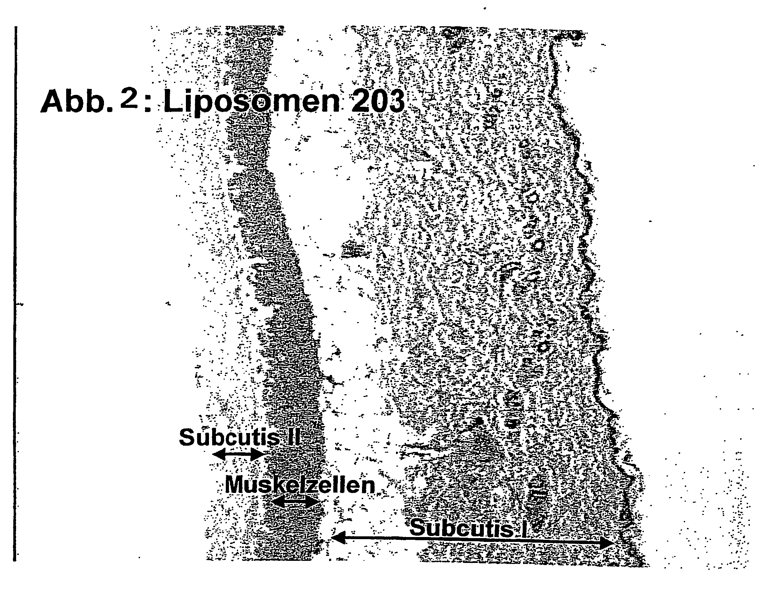

In **Abbildung 1** (Vergrößerung 25fach) ist Haut nach der Applikation von WX-UK1, gelöst in den Liposomen 4, dargestellt. Es sind keine Veränderungen zu normaler Haut **(Abbildung 2)** zu erkennnen.

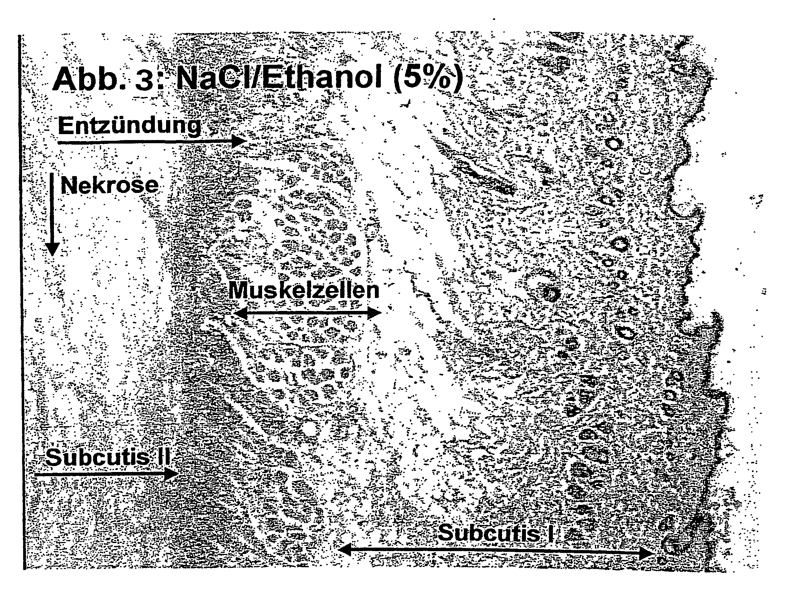

**Abbildung 3** (Vergrößerung 25fach) zeigt den Befund nach Gabe von WX-UK1 in NaCl/Ethanol. Zwischen Subcutis II und den Muskelzellen ist eine starke entzündliche Randreaktion zu erkennen, die Subcutis II ist nekrotisch aufgetrieben.

**Tabelle 2:**

| **Konzentration in Plasma, Galle und Organen nach s.c.-Gabe verschiedener Zubereitungen (n.b. = nicht bestimmt)** | | | | | | | |
|---|---|---|---|---|---|---|---|
| **Vers.- Nr.** | **Gabe** | **Konzentration (µg/ml)** | | **Organgehalt (µg/g)** | | | |
| | | **Plasma** | **Galle** | **Niere** | **Leber** | **Milz** | **Herz** |
| 628 | Kontrolle | <0,05 | 3,1 | 2,5 | 0,36 | 0,65 | 1,0 |
| 629 | Kontrolle | <0,05 | 3,1 | 2,3 | 0,18 | 0,69 | 0,84 |
| | | | | | | | |
| 624 | Liposomen 1 | <0,05 | 2,7 | 1,9 | 0,18 | 0,46 | 0,31 |
| 625 | Liposomen 1 | <0,05 | 4,2 | 1,5 | 0,48 | 0,44 | 0,88 |
| Mittelw. | | <0,05 | 3,4 | 1,7 | 0,33 | 0,45 | 0,60 |
| | | | | | | | |
| 626 | Liposomen 2 | <0,05 | 1,6 | 0,66 | 0,23 | 0,23 | 0,31 |
| 627 | Liposomen 2 | <0,05 | 2,5 | 1,7 | 0,35 | 0,14 | 0,32 |
| Mittelw. | | <0,05 | 2,05 | 1,2 | 0,29 | 0,18 | 0,32 |
| | | | | | | | |
| 632 | Liposomen 3 | n.b. | 7,2 | 4,12 | 0,90 | 0,40 | 1,74 |
| 633 | Liposomen 3 | n.b. | 6,85 | 2,49 | 0,31 | 0,49 | 0,66 |
| Mittelw. | | | 7,0 | ,030 | 0,60 | 0,44 | 1,20 |
| | | | | | | | |
| 637 | Liposomen 4 | n.b. | 6,78 | 2,92 | 0,36 | 0,38 | 1,09 |
| 638 | Liposomen 4 | n.b. | 9,8 | 3,49 | 0,78 | 0,48 | 1,19 |
| Mittelw. | | | 8,3 | 3,20 | 0,57 | 0,43 | 1,50 |

**Tabelle 3:**

| **Histologische Bewertung der untersuchten Hautareale** | | | |
|---|---|---|---|
| **Nr.** | **Gabe** | **Score** | **Befund** |
| 628 | Kontrolle | 3 | umschriebene Nekrosen mit entzündlicher Randreaktion (Leukozyten, beginnendes Granulationsgewebe) |
| 629 | Kontrolle | 3 | wie 629 |
| MW = 3 | | | |
| 624 | Lip. 1 | 0 | ohne Befund/keine Histo |
| 625 | Lip. 1 | 1 | nur 1 Herd: Nekrosen und ausgepr. Randrkt. (wie Kontr.) |
| MW = 0,5 | | | |
| 626 | Lip. 2 | 1 | 2 kleine Herde: entzündliche Reaktionen |
| 627 | Lip. 2 | 0 | normal, geringste entzündliche Infiltrationen |
| MW = 0,5 | | | |
| 632 | Lip. 3 | 1 | 1 Herd: Nekrose mit erheblichen leukozytischen Randreaktion |
| 633 | Lip. 3 | 0 | unauffällig |
| MW = 0,5 | | | |
| 637 | Lip. 4 | 0,5 | keine Nekrose, geringe bandförmige entzündliche Reaktion |
| 638 | Lip. 4 | 0,5 | wie 637 |
| MW = 0,5 | | | |

**Tabelle 4:**

| **Bewertung der Beeinträchtigung der Tiere nach s.c.-Gabe verschiedener Zubereitungen** | | | | | | |
|---|---|---|---|---|---|---|
| **Vers.- Nr.** | **Gabe** | **Verhalten der Tiere** | **Palpation laufend** | **Eindruck Abschluss** | **Histolog. Befund** | **Score** |
| 628 | Kontrolle | 1 | 1 | 2,5 | 3 | 7,5 |
| 629 | Kontrolle | 1 | 1 | 2,5 | 3 | 7,5 |
| Mittelw. = 7,5 | | | | | | |
| 624 | Liposomen 1 | 0 | 0 | 0 | 0 | 0 |
| 625 | Liposomen 1 | 0 | 0,5 | 0,5 | 1 | 2 |
| Mittelw. = 1 | | | | | | |
| 626 | Liposomen 2 | 0 | 0 | 0,5 | 1 | 1,5 |
| 627 | Liposomen 2 | 0 | 0 | 0 | 0 | 0 |
| Mittelw. = 0,75 | | | | | | |
| 632 | Liposomen 3 | 0 | 1 | 0,5 | 1 | 2 |
| 633 | Liposomen 3 | 0 | 0,5 | 0 | 0 | 0,5 |
| Mittelw. = 1,25 | | | | | | |
| 637 | Liposomen 4 | 0 | 1 | 0 | 0,5 | 1,5 |
| 638 | Liposomen 4 | 0 | 1 | 0 | 0,5 | 1,5 |
| Mittelw. = 1,5 | | | | | | |

### Beispiel 3: Verträglichkeit liposomaler Formulierungen im Kaninchen

16 männliche Himalaya-Kaninchen (LPT Labor der Pharmakologie und Toxikologie, Löhndorf) mit einem Körpergewicht zwischen 2,15 und 2,40 kg und einem Alter von etwa 3,5 Monaten wurden als Versuchstiere eingesetzt.

Die Wirkstoffformulierungen wurden subkutan verabreicht. Bei zwei Kaninchen erfolgte die Verabreichung im Bereich des Halses und bei weiteren zwei Kaninchen in die Flanke. Jedes Tier erhielt vier subkutane Injektionen pro Applikationstag in einem Intervall von 2 Tagen an den Untersuchungstagen 1, 3, 5 und 7. Das Volumen der verabreichten Formulierung war zwischen 0,41 und O, 5 ml/Applikation.

Es wurden die in Beispiel 1 beschriebenen liposomalen Formulierungen 1-4 verabreicht. Dabei wurden keinerlei lokale Unverträglichkeitsreaktionen gefunden. Es gab keine Anzeichen für Ödeme, Erytheme, nekrotische Veränderungen oder mechanische Veränderungen (Kratzen).

Auch morphologische Untersuchungen, beginnend 24 h nach der letzten der vier subkutanen Applikationen für insgesamt 4 Tage zeigten keinerlei mit der Wirksubstanz assoziierten morphologischen Veränderungen.

Klinische Anzeichen von systemischer Toxizität wurden nicht gefunden. Das Fressverhalten der Kaninchen war unbeeinflusst. Dermatologische Untersuchungen zeigten keine Veränderungen der Parameter Thromboplastinzeit und aktivierte partielle Thromboplastinzeit.

### Beispiel 4: Pharmakologische Wirksamkeit von liposomalen Formulierungen in der Ratte

Die pharmakologische Wirksamkeit der erfindungsgemäßen liposomalen Formulierungen wurde an weiblichen Ratten (Alter 7 Wochen, Gewicht 100-150 g) getestet.

Es wurden insgesamt 15 Ratten verwendet, die in 5 Gruppen (jeweils 3 Ratten/Gruppe) aufgeteilt wurden. Den Ratten wurden metastasierte Brustkarzinomzellen BN-472 (Kort et al., J. Natl. Cancer Inst. 72 (1984), 709-713) unter das Brust-Fettpolster implantiert. Am Tag 3 nach der Tumorinokulation wurde mit folgendem Behandlungsprotokoll begonnen:
- Gruppe A: Vehikelkontrolle, 1x/Tag s.c.
- Gruppe B: wässrige Lösung des Wirkstoffs (0,3 mg/kg), 1x/Tag s.c.
- Gruppe C: liposomale Formulierung 4 (PC/PG pH 8,4), 0,3 mg Wirkstoff/kg, 1x/Tag s.c.
- Gruppe D: liposomale Formulierung 4 (PC/PG pH 8,4), 1,0 mg Wirkstoff/kg, 1x/Tag s.c.
- Gruppe E: liposomale Formulierung 4 (PC/PG pH 8,4), 3,0 mg Wirkstoff/kg, 1x/Tag s.c.

Es wurde das Auftreten von Tumorfoci in der Lunge, in den axillären Lymphknoten und in den intraperitonialen Lymphknoten sowie das Gewicht der Tumore bestimmt.

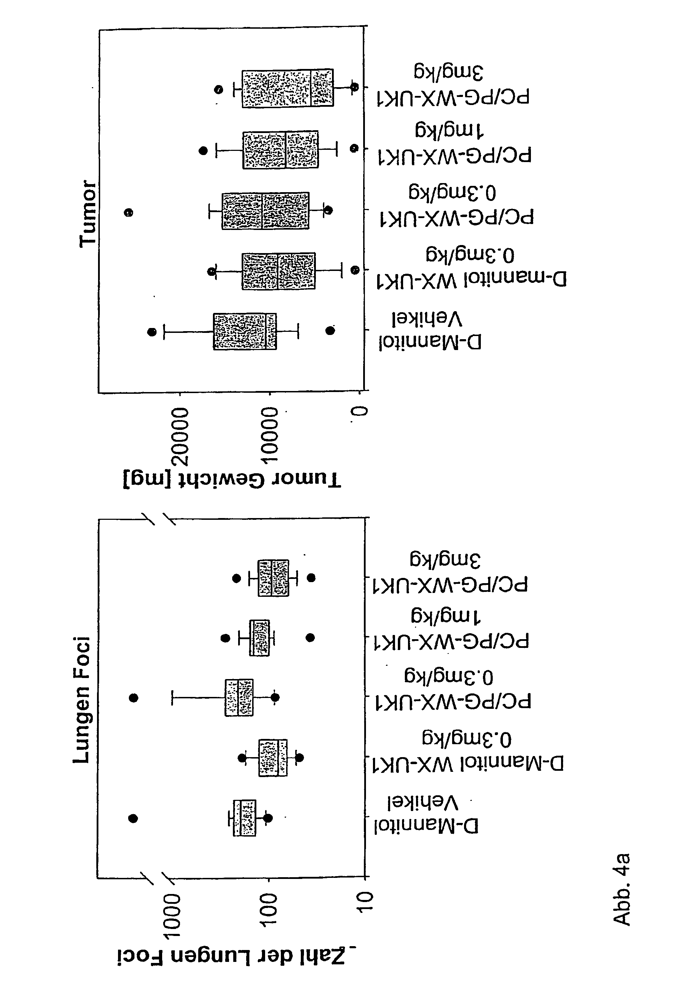

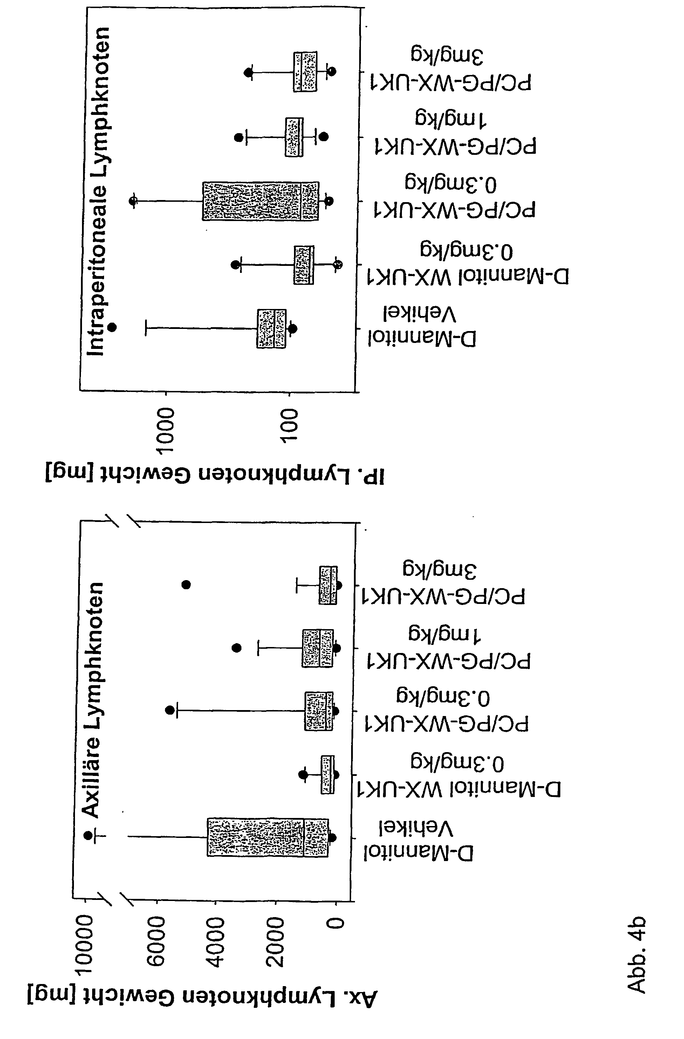

Die Ergebnisse sind in **Abbildung 4** dargestellt und zeigen, dass die liposomale Formulierung pharmakologisch wirksam ist.

### Beispiel 5: Hämolysetest mit liposomalen Formulierungen

Es sollte geprüft werden, ob liposomale WX-UK1-Formulierungen in der Lage sind, hämolytische Eigenschaften des Wirkstoffs zu reduzieren. Der Hämolysetest wurde parallel mit verdünntem Vollblut und gewaschenen Erythrozyten jeweils im Doppelansatz durchgeführt.

1 ml Blut (stabilisiert mit Citrat) wurde mit 4 ml 0,9 %iger NaCl-Lösung verdünnt und in Aliquots von jeweils 200 µl unterteilt. 1 ml der jeweils getesteten Wirkstofflösung (600, 240, 120, 60, 24, 12, 0 µg/ml bezogen auf den Wirkstoffgehalt) wurden zu den 200 µl Blut gegeben, vermischt und bei 37 °C inkubiert. Nach 30 min Inkubation wurde 5 min bei ca. 1.000 xg zentrifugiert. Die Überstände wurden abgenommen und der Hämolysegrad wurde visuell beurteilt.

Zum Hämolysetest an gewaschenen Erythrozyten wurde 1 ml Citrat-Blut mit 5 ml 0,9 %iger NaCl-Lösung mehrmals gewaschen und 0,4 ml der gewaschenen Erythrozyten mit 4,4 ml 0,9 %iger NaCl-Lösung vermischt. Die resultierende Mischung wurde in Aliquots zu jeweils 200 µl aufgeteilt und ansonsten wie Vollblut behandelt.

Als liposomale Formulierungen wurden die Formulierungen 1 (EPC, pH 6,5) und 2 (PC/PG, pH 6,5) gemäß Beispiel 1 getestet. Lediglich bei der höchsten Konzentration (500 µg) der Formulierung 1 wurde eine schwache Hämolyse im Vollblut festgestellt. Alle höheren Verdünnungen waren nicht hämolytisch. Die Formulierung 2 war bei keiner der getesteten Konzentrationen mit Vollblut bzw. gewaschenen Erythrozyten hämolytisch.

Im Vergleich nicht liposomalen Formulierungen wurde eine mindestens 10fache Verringerung der hämolytischen Wirkung für die Formulierung 1 eine noch erheblich höhere Verringerung für die Formulierung 2 gefunden.

### Beispiel 6: Bestimmung der Konzentration von freiem, nicht liposomal komlexierten Wirkstoff WX-UK1 in liposomalen Formulierungen

Es wurden die Formulierungen 1-4 gemäß Beispiel 1 getestet.

60 µl der Formulierung wurden durch eine Microcon YM-50 Filtereinheit zentrifugiert und dann in einer HPLC auf den Gehalt an verbleibendem Wirkstoff mithilfe einer Kalibrierungsgeraden untersucht.

Der Gehalt an freiem WX-UK1 in den entsprechenden Formulierungen beträgt:

| | |
|---|---|
| Formulierung 1 (EPC, pH 6,5) | 550 *µ*g/ml |
| Formulierung 2 (PC/PG, pH 6,5) | 42 *µ*g/ml |
| Formulierung 3 (EPC, pH 8,4) | 461 *µ*g/ml |
| Formulierung 4 (PC/PG, pH 8,4) | 39 *µ*g/ml |

## Patentansprüche

1. Pharmazeutische liposomale Formulierung,
**dadurch gekennzeichnet,**
**dass** sie als Wirkstoff ein als Urokinase-Inhibitor wirksames 3-Amidino- oder 3-Guanidino-phenylalanin-Derivat enthält, wobei der Wirkstoff in einem Gewichtsanteil von 0,5-10 % bezogen auf das Gesamtgewicht der Formulierung vorliegt.

2. Formulierung nach Anspruch 1,
**dadurch gekennzeichnet,**
**dass** der Urokinase-Inhibitor ausgewählt ist aus Nα-(2,4,6-Triisopropylphenylsulfonyl)-3-amidino-(D,L)-phenylalanin-4-ethoxy-carbonylpiperazid, dem L-Enantiomer davon oder einem pharmazeutisch verträglichen Salz dieser Verbindungen.

3. Formulierung nach Anspruch 1,
**dadurch gekennzeichnet,**
**dass** der Urokinase-Inhibitor ausgewählt ist aus Nα-(2,4,6-Triisopropylphenylsulfonyl)-3-guanidino-(D,L)-phenylalanin-4-ethoxy-carbonylpiperazid, dem L-Enantiomer davon oder einem pharmazeutisch verträglichen Salz dieser Verbindungen.

4. Formulierung nach einem der Ansprüche 1 bis 3,
**dadurch gekennzeichnet,**
**dass** der Wirkstoff in einem Gewichtsanteil von 2-5 % vorliegt.

5. Formulierung nach einem der Ansprüche 1 bis 4,
**dadurch gekennzeichnet,**
**dass** sie einen pH-Wert im Bereich von 5,5-9,0 aufweist.

6. Formulierung nach einem der Ansprüche 1 bis 5,
**dadurch gekennzeichnet,**
**dass** sie Phospholipide in einem Gewichtsanteil von 4,5-40 % bezogen auf das Gesamtgewicht der Formulierung enthält.

7. Formulierung nach einem der Ansprüche 1 bis 6,
**dadurch gekennzeichnet,**
**dass** sie Phospholipide, ausgewählt aus neutralen Phospholipiden, anionischen Phospholipiden und Kombinationen davon enthält.

8. Formulierung nach einem der Ansprüche 1 bis 7,
**dadurch gekennzeichnet,**
**dass** sie mindestens ein anionisches Phospholipid, wie etwa Phosphatidylethanolamin, Phosphatidylglycerin, Diphosphatidylglycerin, Phosphoinositol oder veresterte Derivate davon enthält.

9. Formulierung nach Anspruch 7 oder 8,
**dadurch gekennzeichnet,**
**dass** sie Phosphatidylcholin und Dimyristoyl-phosphatidylglycerin in einem Gewichtsverhältnis von 70:30 enthält.

10. Formulierung nach einem der Ansprüche 1 bis 9,
**dadurch gekennzeichnet,**
**dass** sie weiterhin eine membranstabilisierende Komponente, wie etwa Cholesterin, in einem Gewichtsanteil von bis zu 5 % bezogen auf das Gesamtgewicht der Formulierung enthält.

11. Formulierung nach einem der Ansprüche 1 bis 10,
**dadurch gekennzeichnet,**
**dass** sie weiterhin ein Gefrierschutzmittel enthält.

12. Formulierung nach Anspruch 11,
**dadurch gekennzeichnet,**
**dass** das Gefrierschutzmittel in einem Gewichtsanteil von bis zu 15 %, vorzugsweise 5-15 %, bezogen auf das Gesamtgewicht der Formulierung vorliegt.

13. Formulierung nach einem der Ansprüche 11 oder 12,
**dadurch gekennzeichnet,**
**dass** das Gefrierschutzmittel aus Kohlenhydraten oder/und Zuckeralkoholen ausgewählt ist.

14. Formulierung nach einem der Ansprüche 1 bis 13,
**dadurch gekennzeichnet,**
**dass** der mittlere Durchmesser der Liposomen nicht größer als 500 nm ist.

15. Formulierung nach Anspruch 14,
**dadurch gekennzeichnet,**
**dass** der mittlere Durchmesser der Liposomen von 100-250 nm ist.

16. Formulierung nach einem der Ansprüche 1 bis 15,
**dadurch gekennzeichnet,**
**dass** die Liposomen unilamellare Liposomen sind.

17. Formulierung nach einem der Ansprüche 1 bis 16 zur parenteralen Applikation.

18. Formulierung nach Anspruch 17 zur intravenösen Injektion.

19. Formulierung nach Anspruch 17 zur Infusion.

20. Formulierung nach Anspruch 17 zur subkutanen Injektion.

21. Formulierung nach Anspruch 17 zur intramuskulären Injektion.

22. Formulierung nach einem der Ansprüche 1 bis 21 in dehydratisierter Form.

23. Formulierung nach einem der Ansprüche 1 bis 22 zur Bekämpfung von Urokinaseassoziierten Erkrankungen.

24. Formulierung nach Anspruch 23 zur Tumorbekämpfung.

25. Formulierung nach Anspruch 24 zur Bekämpfung von Mammakarzinomen, Pankreaskarzinomen oder/und der Metastasenbildung.

26. Verwendung einer Formulierung nach einem der Ansprüche 1 bis 25 in Kombination mit cytostatischen Mitteln, zur Herstellung eines Arzneimittels.

## Claims

1. Pharmaceutical liposomal formulation, **characterized in that** it comprises as active ingredient a 3-amidino- or 3-guanidino-phenylalanine derivative which is effective as urokinase inhibitor, where the active ingredient is present in a proportion by weight of 0.5-10% based on the total weight of the formulation.

2. Formulation according to Claim 1, **characterized in that** the urokinase inhibitor is selected from Nα-(2,4,6-triisopropylphenylsulphonyl)-3-amidino-(D,L)-phenylalanine 4-ethoxycarbonylpiperazide, the L enantiomer thereof or a pharmaceutically suitable salt of these compounds.

3. Formulation according to Claim 1, **characterized in that** the urokinase inhibitor is selected from Nα-(2,4,6-triisopropylphenylsulphonyl)-3-guanidino-(D,L)-phenylalanine 4-ethoxycarbonylpiperazide, the L enantiomer thereof or a pharmaceutically suitable salt of these compounds.

4. Formulation according to any of Claims 1 to 3, **characterized in that** the active ingredient is present in a proportion by weight of 2-5%.

5. Formulation according to any of Claims 1 to 4, **characterized in that** it has a pH in the range 5.5-9.0.

6. Formulation according to any of Claims 1 to 5, **characterized in that** it comprises phospholipids in a proportion by weight of 4.5-40% based on the total weight of the formulation.

7. Formulation according to any of Claims 1 to 6, **characterized in that** it comprises phospholipids selected from neutral phospholipids, anionic phospholipids and combinations thereof.

8. Formulation according to any of Claims 1 to 7, **characterized in that** it comprises at least one anionic phospholipid such as, for example, phosphatidylethanolamine, phosphatidylglycerol, diphosphatidylglycerol, phosphoinositol or esterified derivatives thereof.

9. Formulation according to Claim 7 or 8, **characterized in that** it comprises phosphatidylcholine and dimyristoylphosphatidyl-glycerol in a ratio of 70:30 by weight.

10. Formulation according to any of Claims 1 to 9, **characterized in that** it additionally comprises a membrane-stabilizing component such as, for example, cholesterol, in a proportion by weight of up to 5% based on the total weight of the formulation.

11. Formulation according to any of Claims 1 to 10, **characterized in that** it additionally comprises a cryoprotectant.

12. Formulation according to Claim 11, **characterized in that** the cryoprotectant is present in a proportion by weight of up to 15%, preferably 5-15%, based on the total weight of the formulation.

13. Formulation according to either of Claims 11 or 12, **characterized in that** the cryoprotectant is selected from carbohydrates or/and sugar alcohols.

14. Formulation according to any of Claims 1 to 13, **characterized in that** the average diameter of liposomes is not greater than 500 nm.

15. Formulation according to Claim 14, **characterized in that** the average diameter of liposomes is of 100-250 nm.

16. Formulation according to any of Claims 1 to 15, **characterized in that** the liposomes are unilamellar liposomes.

17. Formulation according to any of Claims 1 to 16 for parenteral administration.

18. Formulation according to Claim 17 for intravenous injection.

19. Formulation according to Claim 17 for infusion.

20. Formulation according to Claim 17 for subcutaneous injection.

21. Formulation according to Claim 17 for intramuscular injection.

22. Formulation according to any of Claims 1 to 21 in dehydrated form.

23. Formulation according to any of Claims 1 to 22 for controlling urokinase-associated disorders.

24. Formulation according to Claim 23 for controlling tumours.

25. Formulation according to Claim 24 for controlling carcinomas of the breast, pancreatic carcinomas or/and the formation of metastases.

26. Use of a formulation according to any of Claims 1 to 25 in combination with cytostatic agents for the preparation of a medicament.

## Revendications

1. Formulation liposomale pharmaceutique, **caractérisée en ce qu'**elle contient comme substance active un dérivé de 3-amidino- ou 3-guanidino-phénylalanine ayant un effet d'inhibiteur d'urokinase, la substance active étant présente en une fraction en masse de 0,5-10 % par rapport à la masse totale de la formulation.

2. Formulation selon la revendication 1, **caractérisée en ce que** l'inhibiteur d'urokinase est choisi parmi le 4-éthoxycarbonylpipérazide de Nα-(2,4,6-triisopropylphénylsulfonyl)-3-amidino-(D,L)-phénylalanine, son énantiomère L ou un sel pharmaceutiquement acceptable de ces composés.

3. Formulation selon la revendication 1, **caractérisée en ce que** l'inhibiteur d'urokinase est choisi parmi le 4-éthoxycarbonylpipérazide de Nα-(2,4,6-triisopropylphénylsulfonyl)-3-guanidino-(D,L)-phénylalanine, son énantiomère L ou un sel pharmaceutiquement acceptable de ces composés.

4. Formulation selon l'une des revendications 1 à 3, **caractérisé en ce que** la substance active est présente en une fraction en masse de 2-5 %.

5. Formulation selon l'une des revendications 1 à 4, **caractérisée en ce qu'**elle a un pH dans le domaine de 5,5-9,0.

6. Formulation selon l'une des revendications 1 à 5, **caractérisée en ce qu'**elle contient des phospholipides en une fraction en masse de 4,5-40 % par rapport à la masse totale de la formulation.

7. Formulation selon l'une des revendications 1 à 6, **caractérisée en ce qu'**elle contient des phospholipides choisis parmi des phospholipides neutres, des phospholipides anioniques et leurs combinaisons.

8. Formulation selon l'une des revendications 1 à 7, **caractérisée en ce qu'**elle contient au moins un phospholipide anionique comme, par exemple, la phosphatidyléthanolamine, le phosphatidylglycérol, le diphosphatidylglycérol, le phosphoinositol ou leurs dérivés estérifiés.

9. Formulation selon la revendication 7 ou 8, **caractérisée en ce qu'**elle contient de la phosphatidylcholine et du dimyristoyl-phosphatidylglycérol en un rapport en masse de 70:30.

10. Formulation selon l'une des revendications 1 à 9, **caractérisée en ce qu'**elle contient en outre un constituant stabilisant la membrane comme, par exemple, le cholestérol, en une fraction en masse allant jusqu'à 5 % par rapport à la masse totale de la formulation.

11. Formulation selon l'une des revendications 1 à 10, **caractérisée en ce qu'**elle contient en outre un antigel.

12. Formulation selon la revendication 11, **caractérisée en ce que** l'antigel est présent en une fraction en masse allant jusqu'à 15 %, de préférence de 5-15 %, par rapport à la masse totale de la formulation.

13. Formulation selon l'une des revendications 11 ou 12, **caractérisée en ce que** l'antigel est choisi parmi des hydrates de carbone et/ou des alcools de sucres.

14. Formulation selon l'une des revendications 1 à 13, **caractérisée en ce que** le diamètre moyen des liposomes n'est pas supérieur à 500 nm.

15. Formulation selon la revendication 14, **caractérisée en ce que** le diamètre moyen des liposomes est de 100-250 nm.

16. Formulation selon l'une des revendications 1 à 15, **caractérisée en ce que** les liposomes sont des liposomes unilamellaires.

17. Formulation selon l'une des revendications 1 à 16, destinée à l'administration parentérale.

18. Formulation selon la revendication 17, destinée à l'injection intraveineuse.

19. Formulation selon la revendication 17, destinée à la perfusion.

20. Formulation selon la revendication 17, destinée à l'injection sous-cutanée.

21. Formulation selon la revendication 17, destinée à l'injection intramusculaire.

22. Formulation selon l'une des revendications 1 à 21 sous une forme déshydratée.

23. Formulation selon l'une des revendications 1 à 22, destinée à la lutte contre les maladies associées à l'urokinase.

24. Formulation selon la revendication 23, destinée à la lutte contre les tumeurs.

25. Formulation selon la revendication 24, destinée à la lutte contre le cancer du sein, le cancer du pancréas et/ou la formation de métastases.

26. Utilisation d'une formulation selon l'une des revendications 1 à 25 en combinaison avec des agents cytostatiques pour la préparation d'un médicament.
